# EUROPEAN PATENT SPECIFICATION

(11) **EP 1 727 875 B1**
(45) Date of publication and mention of the grant of the patent: **04.11.2015**
(21) Application number: 05726117.4
(22) Date of filing: 24.03.2005
(51) Int. Cl.: C07C 29/145, C07C 29/60, C07C 31/04, C07C 31/20, C07C 45/52, C07C 49/17, C09K 3/18, C09K 5/20, C12C 11/02

(54) **Process for producing acetol from glycerol**
Verfahren zur Herstellung von Acetol aus Glycerin
Procédé de production d'acétol à partir de glycérol

(30) Priority: 25.03.2004 US 556334 P
(43) Date of publication of application: 06.12.2006
(60) Divisional application: 10184100.5
(73) Proprietor: Suppes, Galen J., Columbia MO 65203 (US); The Curators of the University of Missouri, Columbia, MO 65211 (US); Renewable Alternatives LLC, Plymouth, MN 55441 (US)
(72) Inventor: SUPPES, Galen, J., Columbia, MO 65203 (US); SUTTERLIN, William, Rusty, Columbia, MO 65202 (US); DASARI, Mohanprasad, A., Columbia, MO 65203 (US)
(74) Representative: m patent group
(86) International application number: PCT/US2005/009901
(87) International publication number: WO 2005/095536

(56) References cited:
- EP-A- 0 523 015
- EP-A- 0 544 157
- EP-A- 0 826 691
- WO-A-01/46102
- WO-A-01/66499
- WO-A-03/087041
- DE-A1- 4 128 692
- DE-A1- 4 302 464
- DE-C- 524 101
- GB-A- 490 211
- US-A- 5 616 817
- US-A- 5 811 597
- US-A- 6 080 898
- US-A1- 2002 077 501
- DASARI M A ET AL: "Low-pressure hydrogenolysis of glycerol to propylene glycol" APPLIED CATALYSIS A: GENERAL, ELSEVIER SCIENCE, AMSTERDAM, NL, vol. 281, no. 1-2, 1 January 2005 (2005-01-01), pages 225-231, XP004771366 ISSN: 0926-860X
- MONTASSIER C ET AL: "Deactivation of supported copper based catalysts during polyol conversion in aqueous phase" APPLIED CATALYSIS A: GENERAL, ELSEVIER SCIENCE, AMSTERDAM, NL, vol. 121, no. 2, 19 January 1995 (1995-01-19), pages 231-244, XP004300293 ISSN: 0926-860X
- MONTASSIER C ET AL: "POLYOL CONVERSION BY LIQUID PHASE HETEROGENEOUS CATALYSIS OVER METALS" HETEROGENEOUS CATALYSIS AND FINE CHEMICALS, 1988, pages 165-170, XP009053599
- DATABASE WPI Section Ch, Week 200316 Derwent Publications Ltd., London, GB; Class D23, AN 2003-159935 XP002361759 -& JP 2002 265986 A (KOBAYASHI A) 18 September 2002 (2002-09-18)

## Description

### BACKGROUND

### 1. Field of The Invention

This invention relates generally to a process for value-added processing of fats and oils to yield glycerol and glycerol derivatives. More particularly, the process converts glycerol to acetol and/or propylene glycol, which is also known as 1, 2 propanediol. The process may yield glycerol-based products and glycerol derivatives, such as antifreeze and other products.

### 2. Description of The Related Art

Conventional processing of natural glycerol to propanediols uses a catalyst, for example, as reported in United States patents 5,616,817, 4,642,394, 5,214,219 and US 5,276,181. These patents report the successful hydrogenation of glycerol to form propanediols. None of the processes shown by these patents provide a direct reaction product mixture that is suitable for use as antifreeze. None provide process conditions and reactions that suitably optimize the resultant reaction product mixture for direct use as antifreeze. None address the use of unrefined crude natural glycerol feed stock, and none of these processes are based on reactive distillation.

United States patent 5,616,817 issued to Schuster et al. describes the catalytic hydrogenation of glycerol to produce propylene glycol in high yield, such as a 92% yield, with associated formation of n-propanol and lower alcohols. Conversion of glycerol is substantially complete using a mixed catalyst of cobalt, copper, manganese, and molybdenum. Hydrogenation conditions include a pressure of from 100 to 700 bar and a temperature ranging from 180°C to 270°C. Preferred process conditions include a pressure of from 200 to 325 bar and a temperature of from 200°C to 250°C. The lower pressures lead to incomplete reactions and the higher pressures increasingly form short chain alcohols. A crude glycerol feed may be used, such as is obtainable from the transesterification of fats and oils, but needs to be refined by short path distillation to remove contaminants, such as sulfuric acid that is commonly utilized in the transesterification process. The feed should contain glycerol in high purity with not more than 20% water by weight.

United States patent 4,642,394 issued to Che et al. describes a process for catalytic hydrogenation of glycerol using a catalyst that contains tungsten and a Group VIII metal. Process conditions include a pressure ranging from 100 psi to 15,000 psi and a temperature ranging from 75°C to 250°C. Preferred process conditions include a temperature ranging from 100°C to 200°C and a pressure ranging from 200 to 10,000 psi. The reaction uses basic reaction conditions, such as may be provided by an amine or amide solvent, a metal hydroxide, a metal carbonate, or a quaternary ammonium compound. The concentration of solvent may be from 5 to 100 ml solvent per gram of glycerol. Carbon monoxide is used to stabilize and activate the catalyst. The working examples show that process yields may be altered by using different catalysts, for example, where the yield of propanediols may be adjusted from 0% to 36% based upon the reported weight of glycerol reagent.

United States patents 5,214,219 issued to Casale, et al. and 5,276,181 issued to Matsumura, et al. describe the catalytic hydrogenation of glycerol using a copper/zinc catalyst. Process conditions include a pressure ranging from 5 MPa to 20 MPa and a temperature greater than 200 C. Preferred process conditions include a pressure ranging from 10 to 15 MPa and a temperature ranging from 220°C to 280°C. The concentration of glycerol may range from 20% to 60% by weight in water or alcohol, and this is preferably from 30% to 40% by weight. The reaction may be adjusted to produce significant amounts of hydrocarbon gas and/or lactic acid, such that gas generation is high when lactic acid formation is low and lactic acid formation is high when gas generation is low. This difference is a function of the amount of base, i.e., sodium hydroxide, which is added to the solvent. Alcohol reaction products may range from 0% to 13% of hydrocarbon products in the reaction mixture by molar percentages, and propanediols from 27% to 80%. Glycerol conversion efficiency ranges from 6% to 100%.

M.A. Dasari et al.: "Low-pressure hydrogenolysis of glycerol to propylene glycol", Applied Catalysis A: General 281 (2005) 225-231, discloses a method for the hydrogenolysis of glycerol to propylene glycol using nickel, palladium, platinum, copper and cop-per-chromite catalysts. The effects of temperature, hydrogen pressure, initial water content, choice of catalyst, catalyst reduction temperature and the amount of catalyst are evaluated.

In DE 41 28 692 A1, a method of producing acetol via dehydration of glycerol at higher temperatures is disclosed. It is proposed to react glycerol with a heterogenous catalyst comprising an element of the first and/or eighth subgroup at temperatures between 180 and 400°C to acetol and water.

### SUMMARY

According to the present invention, a process for converting glycerol to acetol with high selectivity is provided, comprising the steps of combining a glycerol-containing feed-stock with less than 50% by weight water with a catalyst that is capable of dehydrating glycerol to form a reaction mixture, heating the reaction mixture to a temperature ranging from 170°C to 270° C over a reaction time of up to 24 hours at a pressure ranging from 0.2 to 25 bar, and using hydrogen as a stripper gas to strip the acetol from solution. Preferred embodiments are subject of the dependent claims and of the description that follows.

The presently disclosed process advances the art and overcomes the problems outlined above by producing value-added products, such as antifreeze; from hydrogenation of natural glycerol feed stocks.

As the reaction, according to the present invention, is limited by hydrogen, an acetol product is formed.

This reaction product may be hydrogenated in a further reaction step to produce propylene glycol.

Particularly preferred glycerol-containing feedstocks include those that are produced from bio-renewable resources, such as vegetable oils and especially soy oil. The feedstock may, for example, be provided as the crude glycerol byproduct of a C₁ to C₄ alkyl alcohol alcoholysis of a glyceride.

As the process for converting glycerol to propylene glycol is interrupted by limiting the hydrogen reagent, it results in the production of acetol. This product may be provided as a feedstock for a further catalyzed reaction with hydrogen to complete the conversion to propylene glycol. The process of converting acetol or lactaldehyde to propylene glycol has high selectivity.

### BRIEF DESCRIPTION OF DRAWINGS

**Fig. 1** is a schematic block flow diagram illustrating a reactor- separator with a reactor, condenser, and condensate tank, and recycle of unreacted hydrogen.
**Fig. 2** is a schematic of the reaction mechanism for conversion of glycerol to propylene glycol via acetol intermediate.
**Fig. 3** is a schematic of the reaction mechanism for converting glycerol to acetol and then converting acetol to propylene glycol.
**Fig. 4** is a schematic of the reaction mechanism for conversion of acetol to propylene glycol via lactaldehyde intermediate.
**Fig. 5** is a schematic of the proposed two-step embodiment for converting glycerol to acetol and then converting acetol to propylene glycol where hydrogen is used for the first reactor at a lower pressure and then the hydrogen is compressed for use in the second reactor.
**Fig. 6** is a schematic of the proposed two-step alternative embodiment for converting glycerol to acetol and then converting acetol to propylene glycol where hydrogen is used for the first reactor at a lower pressure and water is removed from the vapor effluents from the first reactor to allow purging of the water from the system.

### DETAILED DESCRIPTION OF PREFERRED EMBODIMENTS AND COMPARATIVE EXAMPLES NOT FORMING PART OF THE INVENTION

There will now be shown and described by way of example, and to explain the background of the invention but not forming part thereof, a process for producing lower alcohols from glycerol feed stock. According to the invention, the lower alcohol acetol is produced. Preferred uses of reaction product mixtures that are derived from the inventive and non-inventive processes include deicing fluids, anti-icing fluids, and antifreeze applications.

These uses of the glycerol-based and/or propylene glycol-based antifreezes displace the use of toxic and non-renewable ethylene glycol with non-toxic and renewable glycerol-derived antifreeze. In this regard, use of propylene glycol that is derived from natural glycerol is a renewable alternative to petroleum-derived propylene glycol. Other downstream uses for propylene glycol include any substitution or replacement of ethylene glycol or glycerol with propylene glycol.

### Equipment For Reactive-Separation Preparation of Antifreeze From PolyAlcohols Like Glycerol (Background of the Invention)

One method of preparing antifreeze from glycerol, not forming part of the invention, includes reaction at a temperature ranging from 150°to 250°C and in some embodiments this temperature is more preferably from 180°C to 220°C. The reaction occurs in a reaction vessel. The pressures in the reaction vessel are preferably from 1 to 25 atmospheres (or from 1 to 25 bar) and in some embodiments this pressure is more-preferably between 5 and 18 atmospheres. The process equipment may include, for example, a reactor at these temperature and pressure conditions connected to a condenser and condensate tank where the condenser is preferably at a temperature between about 25°C and 150°C and in some embodiments this is more preferably between 25°and 60°C.

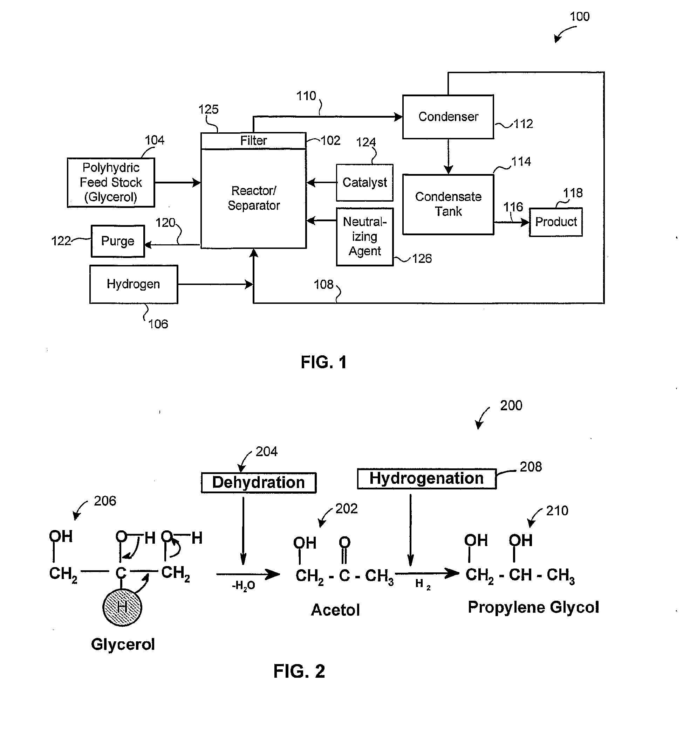

Fig. 1 provides a block flow diagram of process equipment 100 including a reactor-separator 102. A polyhydric feed stock 104, for example, containing glycerol, is introduced stepwise or continuously into the reactor separator 102. Hydrogen 106 is added to hydrogen line 108 to promote conversion of glycerol 104 to propylene glycol within the reactor-separator 102. The process temperatures are such that a distillation occurs with the formation or presence of propylene glycol, short chain alcohols, and water, which vaporize and flow through overhead line 110 to a condenser 112. Most of the alcohol, water and propylene glycol vapors condense in the condenser 112 and are collected in the condensate tank 114 for discharge through discharge line 116 as product 118. Unreacted hydrogen and remaining vapors from the condenser 112 are recycled back to the reactor-separator 102 through the hydrogen recycle line 108.

Reaction products 118 are removed from the condensate tank 112 through discharge line 116, and the reaction mixture inside reactor-separator 102 may be purged periodically or at a slow flow rate through purge line 120 to obtain purge mixture 122. Purging is necessary or desirable when non-volatile reaction by-products are formed and when metals or inorganic acids, such as residual biodiesel catalysts, are present in the polyhydric feed stock 104. Catalysts and useful components, such as glycerol and propylene glycol, are preferably recovered from the purge mixture 122.

The reaction inside reactor-separator 102 is catalyzed, and may be facilitated at periodic intervals or by the continuous introduction of a suitable catalyst 124, which may be any catalyst that is suitable for use in converting glycerol into lower alcohols, such as acetol and/or propylene glycol. The catalyst 124 may reside within the reactor-separator as a packed bed, or distribution of the catalyst 124 inside reactor-separator 102 may be improved by using the hydrogen gas 108 to provide a fluidized bed, or by stirring (not shown). Agitated slurry reactors of a liquid phase reaction with a vapor overhead product are preferred. The catalyst 124 is mixed with the polyhydric feedstock 104 that is undergoing reaction in the reactor separator 102 to facilitate breaking of carbon-oxygen or carbon-carbon bonds including but not limited to hydrogenation. As used herein, hydrogenolysis and hydrogenation are interchangeable terms. By way of example the reaction of glycerol with hydrogen to form propylene glycol and water is referred to frequently as hydrogenation in this text. Suitable catalysts for this purpose may include, without limitation, such metals as platinum, palladium, ruthenium, chromium, nickel, copper, zinc, rhodium, chromium, ruthenium, and combinations thereof. Catalysts may be deposited on a substrate, such as an alumina substrate. The best catalysts are non-volatile, and are preferably prevented from exiting the reactor separator 102 into the condensate tank 114. A filter 125 in the overhead discharge line 110 from the reactor separator 102 retains solid catalysts in the reactor separator 102. No limitations are placed or implied on whether the catalyst is soluble or solid, the oxidative state of the catalyst, or the use of solid supports or soluble chelates.

Reaction times at preferred conditions may range from 2 hours to 96 hours, and this is more preferably from 4 to 28 hours. Reaction time may be defined as the volume of liquid in the reactor divided by the time-averaged flow rate of liquids into the reactor. While the preferred reaction times are greater than 2 hours, the average residence time at higher loadings of catalyst 124 can be less than an hour and typically longer than 0.5 hours. While preferred temperatures are up to 250°C, the reactor-separator may be operated at temperatures up to 270°C with satisfactory results.

The polyhydric feed stock 104 preferably contains glycerol. In a broader sense, polyhydric feedstock 104 may contain, for example, from 5% to substantially 100% of a polyol, for example, glycerol, sorbitol, 6-carbon sugars, 12-carbon sugars, starches and/or cellulose.

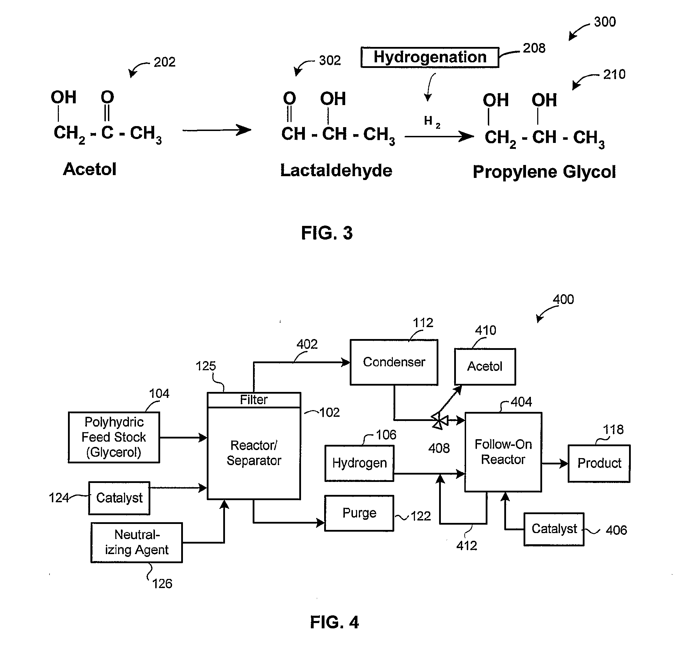

As illustrated in Fig. 1, the process equipment 100 is preferably configured to provide hydrogen 106 as a reagent; however, the use of hydrogen is optional. Commercially valuable products may be formed as intermediates that collect in the condensate tank in the absence of hydrogen. Accordingly, use of hydrogen 106 is preferred, but not necessary. For example, the intermediates collecting in condensate tank 114 may include acetol (hydroxy-2-propanone), which may be subjected to hydrogenolysis in another process, which is shown below in Fig. 3. In addition to reagents, the material within reactor separator 102 may contain water, salts, or catalysts residue from previous processes.

One type of polyhydric feedstock 104 may contain glycerol that is prepared by transesterification of oils or fatty acids, for example, as described in co-pending application serial number 10/420,047 filed April 23, 2003.

In a polyhydric feedstock 104 of this type, water may be present in an amount ranging from 0% to 70%. More preferably, water is present in an amount ranging from 5% to 15%. Water may be added to reduce side-reactions, such as the formation of oligomers.

One advantage of using the process equipment 100 is that volatile alcohol products are removed from the reaction mixture as they are formed inside reactor separator 102. The possibility of degrading these products by continuing exposure to the reaction conditions is commensurately decreased by virtue of this removal. In addition, the volatile reaction products are inherently removed from the catalysts to provide relatively clean products. This reaction-separation technique is especially advantageous for catalysts that are soluble with or emulsified in the reaction mixture.

A preferred class of catalyst 124 is the copper chromite catalyst, (CuO)ₓ(Cr₂O₃)_{y}. This type of catalyst is useful in the process and is generally available commercially. In this class of catalyst, the nominal compositions of copper expressed as CuO and chromium expressed as Cr₂O₃ may vary from about 30-80 wt% of CuO and 20-60 wt% of Cr₂O₃. Catalyst compositions containing about 40-60 wt% copper and 40-50 wt% of chromium are preferred.

Preferred catalysts for use as catalyst 124, in addition to the copper and chromium previously described, also include barium oxide and manganese oxide or any of their combinations. Use of barium and manganese is known to increase the stability of the catalyst, i.e., the effective catalyst life. The nominal compositions for barium expressed as barium oxide can vary from 0-20 wt% and that for manganese expressed as manganese oxide can vary from 0-10 wt%. The most preferred catalyst compositions comprise from 40%-60 wt% of CuO 40-55 wt % of Cr203, 0-10 wt% of barium oxide and 0-5 wt% manganese oxide.

### Reaction Mechanism (Background of the invention)

According to one mechanism proposed by Montassier et al., Heterogenous Catalysis and Fine Chemicals, p. 165-170 (1988), dehydrogenation of glycerol on copper can form glyceric aldehyde in equilibrium with its enolic tautomer. The formation of propylene glycol was explained by a nucleophilic reaction of water or adsorbed OH species, a dehydroxylation reaction, followed by hydrogenation of the intermediate unsaturated aldehyde. This reaction mechanism was observed not to apply in our investigation.

Fig. 2 shows a reaction mechanism 200 for use in the reactor-separator 102 of Fig. 1, and for which process conditions may be suitably adjusted as described above. As shown in Fig. 2, hydroxyacetone (acetol) 202 is formed, and this is possibly an intermediate of an alternative path for forming propylene glycol by a different mechanism. The acetol 202 is formed by dehydration 204 of a glycerol molecule 206 that undergoes intramolecular rearrangements as shown. In a subsequent hydrogenation step 208, the acetol 202 further reacts with hydrogen to form propylene glycol 210 with one mole of water by-product resulting from the dehydration step 204.

Early studies to investigate the effect of water on the hydrogenolysis reaction indicated that the reaction takes place even in absence of water with a 49.7% yield of propylene glycol. Moreover, and by way of example, the reaction is facilitated by use of a copper-chromite catalyst, which may be reduced in a stream of hydrogen prior to the reaction. In this case, the incidence of surface hydroxyl species taking part in the reaction is eliminated. The above observations contradict the mechanism proposed by Montassier et al. where water is present in the form of surface hydroxyl species or as a part of reactants.

### COMPARATIVE EXAMPLE 1

### Confirmation of Reaction Mechanism

An experiment was performed to validate the reaction mechanism 200. Reactions were conducted in two steps, namely, Steps 1 and 2. In step 1, relatively pure acetol was isolated from glycerol. Temperature ranged from 150°C to 250°C and more specifically from 180°C to 220°C. There wa s an absence of hydrogen. Pressure ranged from 1 to 14 psi (6.9 MPa to 96 MPa) more specifically from 5 to 10 psi (34 MPa to 69 MPa). A copper-chromite catalyst was present. In Step 2, the acetol formed in Step 1 was further reacted in presence of hydrogen to form propylene glycol at a temperature ranging from 150°C to 250°C and more preferably between 180°to 220°C. Excess hydrogen was added at a hydrogen over pressure between 1 to 25 bars using the same catalyst.

It was observed in the Step 2 of converting acetol to propylene glycol that lactaldehyde was formed. Propylene glycol is also formed by the hydrogenation 208 of lactaldehyde 302, as illustrated in Fig. 3. With respect to Fig. 2, lactaldehyde represents an alternative path for forming propylene glycol from acetol. Fig. 3 shows this mechanism 300 where the acetol undergoes a rearrangement of the oxygen double bond to form lactaldehyde 302, but the dehydrogenation step 208 acting upon the lactaldehyde 302 also results in the formation of propylene glycol 210. It was also observed that the formation of lactaldehyde intermediate was predominant at lower reaction temperatures in the range of from 50°C to 150°C (see Example 8 below).

This and subsequent reactions were performed in liquid phases with catalyst and sufficient agitation to create a slurry reaction mixture.

### COMPARATIVE EXAMPLE 2

### Simultaneous Dehydration and Hydrogenation Using Various Catalysts and Reagent Mixtures

A variety of reaction procedures were performed to show that reaction efficiency may be optimized at any process conditions, such as reaction time, temperature, pressure and flash condition by the selection or choice of catalyst for a given polyhydric feedstock.

Table 1 reports the results of reacting glycerol in the presence of hydrogen and catalyst to form a mixture containing propylene glycol. The reaction vessel contained 80 grams of refined glycerol, 20 grams of water, 10 grams of catalyst, and a hydrogen overpressure of 13.8 bar (200 psig). The reactor was a closed reactor that was topped off with excess hydrogen. The reaction occurred for 24 hours at a temperature of 200 C. All catalysts used in this

Example were purchased on commercial order and used in the condition in which they arrived.

**Table 1. Summary of catalyst performances based on 80 grams of glycerol reported on a 100 grams basis.**

| | Initial Loading (g) | Best Possible (g) | Catalyst 5% Rutheniu m on carbon (g) | Catalyst Raney-Copper (g) | Catalyst Raney-Nickel (g) |
|---|---|---|---|---|---|
| Glycerol | 100 | 0 | 63.2 | 20.6 | 53.6 |
| Water | 25 | 43 | not measured | not measured | not measured |
| Propylene Glycol | 0 | 82 | 14.9 | 27.5 | 14.9 |
| Ethylene Glycol | 0 | 0 | 16.9 | 13.1 | 16.5 |
| Acetol | 0 | 0 | 0.0 | 12.1 | 0.0 |
| Total, excluding water | 100 | 82 | 94.9 | 73.2 | 85.0 |

Table 2 summarizes reaction performance with a higher initial water content, namely, 30 grams of refined glycerol and 70 grams of water. The reactions were conducted at the following initial conditions: 5% wt of catalyst, and a hydrogen overpressure of 1400 kPa. The following table presents compositions after reacting in a closed reactor (with topping off of hydrogen) for 24 hours at a reaction temperature of 200 C.

**Table 2. Summary of catalyst performances based on 30 grams initial loading of glycerol and 70 grams of water.**

| | Initial Loading (g) | Best Possible (g) | Catalyst 5% Ruthenium on carbon (g) | Catalyst Raney-Copper (g) | Catalyst Raney-Nickel (g) |
|---|---|---|---|---|---|
| Glycerol | 30 | 0 | 20.8 | 19.1 | 3.8 |
| Propylene Glycol | 0 | 24 | 9.3 | 7.23 | 3.1 |
| Ethylene Glycol | 0 | 0 | 0 | 0 | 0 |
| Acetol | 0 | 0 | 1.5 | 1.6 | 1.7 |

Table 3 summarizes the performance of a copper chromium catalyst in the presence of 20 percent of water. The reactions were conducted at the following initial conditions: 5% wt of catalyst, and a hydrogen overpressure of 1400 kPa. The following table presents compositions after reacting in a closed reactor (with topping off of hydrogen) for 24 hours at a reaction temperature of 200°C.

**Table 3. Summary of copper chromium catalyst performance based on 80 grams initial loading of glycerol and 20 grams of water.**

| | Initial Loading (g) | Best Possible (g) | Catalyst Copper Chromium (g) |
|---|---|---|---|
| Glycerol | 80 | 0 | 33.1 |
| Propylene glycol | 0 | 66.1 | 44.8 |
| Ethylene Glycol | 0 | 0 | 0 |
| Acetol | 0 | 0 | 3.2 |

Table 4 summarizes the impact of initial water content in the reactants on formation of propylene glycol from glycerol. The reactions were conducted at the following initial conditions: 5% wt of catalyst, and a hydrogen overpressure of 1400 kPa. The catalyst was purchased from Sud-Chemie as a powder catalyst having 30 m²/g surface area, 45% CuO, 47% Cr₂O₃, 3.5% MnO₂ and 2.7% BaO. The following table presents compositions after reacting in a closed reactor (with topping off of hydrogen) for 24 hours at a reaction temperature of 200°C.

| **Table 4:** Summary of catalyst performances based on different initial loadings of glycerol in water. | | | |
|---|---|---|---|
| Water (wt%) | %Conversion | %Yield | %Selectivity |
| 80 | 33.5 | 21.7 | 64.8 |
| 40 | 48 | 28.5 | 59.4 |
| 20 | 54.8 | 46.6 | 85.0 |
| 10 | 58.8 | 47.2 | 80.3 |
| 0 | 69.1 | 49.7 | 71.9 |

The reaction was performed using a small scale reaction distillation system like that shown as process equipment 100 in Fig. 1 to process a reaction mixture including 46.5 grams of refined glycerol and 53.5 grams water. The catalyst was purchased from Sud-Chemie as a powder catalyst having 30 m²/g surface area, 45% CuO, 47% Cr₂O₃, 3.5% MnO₂ and 2.7% BaO. Table 5 summarizes performance with higher initial water content using a small reaction distillation system.

**Table 5. Example of reaction distillation.**

| | Reactor | Distillate |
|---|---|---|
| Glycerol | 21.6 grams | 2.2 |
| Propane Diol | 6.4 | 9.5 |
| Ethylene Glycol | 0 | 0 |
| Acetol | 1.4 | 1.4 |

### Composition of Antifreeze Product from Glycerol of Biodiesel Facility

Biodiesel is one type of product that can be produced from a fatty acid glycerin refinery. After a conventional biodiesel methanolysis reaction, the methoxylation catalyst is preferably removed by filtration from a slurry reaction system. Other methods, such as centrifugation or precipitation, may be used to remove soluble catalysts from the glycerol by-product of the biodiesel methanolysis reaction process. These processes are compatible with either batch or continuous operation. Methods known in the art may be used to convert the batch process procedures (described herein) to flow process procedures. Hydrogenation of the glycerol is performed to prepare a glycerol byproduct that preferably contains, on a water-free basis, from 0.5% to 60% glycerol, and 20% to 85% propylene glycol. More preferably, the glycerol byproduct contains on a water-free basis from 10% to 35% glycerol, and 40% to 75% propylene glycol. Also, as the preferred antifreeze of this invention is prepared from the crude natural glycerol byproduct of the C₁ to C₄ alkyl alcohol alcoholysis of a glyceride, the more preferable product also contains 0.2% to 10% C₁ to C₄ alkyl alcohol and 0 to 5% salt of the neutralized alcoholysis catalyst (more preferably 0.2 to 5% salt).

The glycerol conversion reactions have been observed to form a residue by-product. When this residue is soluble in the antifreeze product, the preferred application is to add it to the antifreeze product. The antifreeze may contain 1 % to 15% of this residue by-product.

While the antifreeze products of this invention are commonly referred to as antifreeze, these same mixtures or variations thereof may be used as deicing fluids and anti-icing fluids.

When the reaction is run without hydrogen, acetol will form. This mixture can then subsequently (or in parallel) react in a packed-bed flow reactor in the presence of hydrogen to be converted to propylene glycol. This process has the advantage that the larger reactor does not contain pressurized hydrogen.

The processes and procedures described in this text are generally applicable to refined glycerol as well as crude glycerol.

The catalyst used for most of the process development was a Sud-Chemie powder catalyst at 30 m²/g surface area, 45% CuO, 47% Cr₂O₃, 3.5% MnO₂ and 2.7% BaO. Also used was a Sud-Chemie tablet catalyst at 49% CuO, 35% Cr₂O₃, 10% SiO₂ and 6% BaO. Also used was a Sud-Chemie powder catalyst at 54% CuO .

### COMPARATIVE EXAMPLE 3

### Processing of Biodiesel Byproduct

Crude glycerol obtained as a by product of the biodiesel industry was used instead of refined glycerol. Biodiesel is produced using alcoholysis of bio-renewable fats and oils. The composition of feedstock 104 used in this example had an approximate composition as follows: glycerol (57%), methyl alcohol (23%), and other materials (soaps, residual salts, water) (20%). The above feedstock was reacted in the presence of hydrogen and catalyst to form a mixture containing propylene glycol. The reaction proceeded using 10 grams of the crude feedstock, 5% by weight of catalyst, and a hydrogen overpressure of 1400 kPa. The following Table 6 presents compositions after reacting in a closed reactor (with topping off of hydrogen) for 24 hours at a temperature of 200°C. The copper chromium catalyst used in this Example was reduced in presence of hydrogen at a temperature of 300°C for 4 hours prior to the reaction.

| **Table 6:** Summary of catalyst performances based on 10 grams of crude glycerol. | | | |
|---|---|---|---|
| | Initial Loading (g) | Best Possible (g) | Final Product (g) |
| Crude Glycerol | 5.7 | 0 | 0.8 |
| Acetol | 0 | 0 | 0 |
| Propylene glycol | 0 | 4.6 | 3.1 |
| Water | 1 | 2.1 | 2.6 |

### Reactive-Separation To Prepare Acetol and Other Alcohols

As an alternative to reacting to form propylene glycol by use of the process equipment 100 shown in Fig. 1, Fig. 4 shows a modified version of the process equipment that has been previously described. Process equipment 400 is useful for forming acetol or other alcohols having boiling points less than about 200°C. Dehydration is the preferred reaction method, but cracking reactions may be used with feed stocks containing sugars or polysaccharides having carbon numbers greater than 3.

In general, the process equipment 400 is used for converting a three-carbon or greater sugar or polysaccharide to an alcohol dehydration product having a boiling point less than about 200° C. By way of example, a sugar or polysaccharide-containing feedstock with less than 50% by weight water is combined with a catalyst that is capable of dehydrating glycerol to form a reaction mixture. The reaction mixture is heated to a temperature ranging from 170° to 270°C over a reaction time interval ranging from 0.5 to 24 hours at a pressure ranging from 0.2 to 25 bar.

The preferred reaction conditions for conversion of glycerol to form acetol include a process temperature ranging from 170°C to 270°C, and this is more preferably from 180°C to 240°C. The preferred reaction time ranges from 0.5 to 24 hours. Heterogeneous catalysts that are known to be effective for dehydration may be used, such as nickel, copper, zinc, copper chromium, activated alumina and others known in the art. The preferred reaction pressure ranges from 0.2 to 25 bar, and more preferably from 0.5 to 3 bar. The feedstock may contain from 0% to 50% and more preferably 0 to 15% water by weight.

By these instrumentalities, glycerol may be dehydrated to acetol. Selective formation of acetol is documented for the copper-chromium catalyst by Examples 5 through 7 below. The same reaction conditions with different catalyst are effective for forming other alcohol products where the products have fewer alcohol functional groups than do the reagents. Fractional isolation of intermediates through reactive-distillation is particularly effective to increase yields and the embodiments is inclusive of processes to produce a range of products including but not limited to 1,3 propanediol and acrolein.

Fig. 4 shows process equipment 400 for the selective conversion of glycol to acetol. In Fig. 4, identical numbering is used for the same components that have been previously described with respect to Fig. 1. The reactor separator 102 as shown in Fig. 4 functions as a dehydration reactor.

The polyhydric feedstock 104 and catalyst 124 enter reactor-separator 102 for a reaction that is limited to the dehydration step 204 of Fig. 2 by the absence of hydrogen, and in consequence the hydrogenation step 208 does not occur at this time. The dominant reaction product is acetol 202. Volatile fractions including acetol vapor exit the reactor-separator 102 through an overhead intermediate line 402 and liquefy in condenser 112. A follow-on reactor 404 functions as a hydrogenolysis reactor that accepts acetol and other liquids from condenser 112, and contacts the acetol with hydrogen to form propylene glycol as product 118. The catalyst 406 may be the same as or different from catalyst 126. The condenser 112 preferably operates at a temperature ranging from 25°C to 150°C and this is more-preferably from 25°C to 60°C. It will be appreciated that the condenser 112 may be eliminated or positioned downstream of the follow-on reactor 404 if the follow-on reactor 404 operates as a vapor phase reactor.

When the process equipment 400 is operating in mode of producing propylene glycol product 118, a hydrogen recycle loop 412 recycles excess hydrogen from the follow-on reactor 404. This step preferably recycles unused hydrogen from the condenser back to the subsequent step reaction mixture. The reaction time of this subsequent step reaction ranges from 0.2 to 24 hours and more-preferably ranges from 1 to 6 hours.

The acetol that is delivered through intermediate line 402 to condenser 112 is optimally diverted through three way valve 408 to provide an acetol product 410.

### COMPARATIVE EXAMPLE 4

### Stepwise Production of Acetol Then Propylene Glycol

Glycerol was reacted in the presence of copper chromium catalyst in two steps to form a mixture containing propylene glycol. In Step 1, relatively pure acetol was isolated from glycerol in absence of hydrogen at a reaction pressure of 98 kPa (vac). In Step 2, the acetol from Step 1 was further reacted in presence of hydrogen to propylene glycol at 1400 kPa hydrogen over pressure using similar catalyst that is used for the formation of acetol. The catalyst used in the step 1 of this Example is used in the condition in which they arrived and the catalyst used in the Step 2 was reduced in presence of hydrogen at a temperature of 300°C for 4 hours prior to the reaction.

The following tables present composition of the final product in Step 1 and Step 2

| **Table 7.** Example reaction conditions for converting glycerol to propylene glycol. | | | |
|---|---|---|---|
| **Step 1:** Formation and isolation of acetol intermediate from glycerol using copper-chromite catalyst. Catalyst - 5% unreduced powder Cu/Cr, Reaction time - 1.5 hr at 220°C and 3hr at 240°C, Reaction Pressure - 98 kPa (vac). | | | |
| | Initial Loading (g) | Best Possible (g) | Final Product (g) |
| Glycerol | 36.8 | 0 | 3.6 |
| Acetol | 0 | 29.6 | 23.7 |
| Propylene glycol | 0 | 0 | 1.7 |
| Water | 0 | 7.2 | 6.9 |
| | | | |

| **Step 2:** Formation of propylene glycol from acetol intermediate from Step 1 using same catalyst. Catalyst - 5% reduced powder Cu/Cr, Reaction time - 12 hr, Reaction Temperature - 190°C, Reaction Pressure - 1400 kPa. | | | |
|---|---|---|---|
| | Initial Loading (g) | Best Possible (g) | Final Product (g) |
| Glycerol | 0 | 0 | 0 |
| Acetol | 4.5 | 0 | 0 |
| Propylene glycol | 0 | 4.6 | 4.3 |

### COMPARATIVE EXAMPLE 5

### Batch Versus Semi Batch Processing

Glycerol was reacted in presence of copper chromium catalyst to form acetol by each of two process modes: batch and semi batch. Relatively pure acetol was isolated from glycerol in absence of hydrogen at a reaction pressure of 98 kPa (vac). In this reaction 92 grams of glycerol would form a maximum of 74 grams acetol at the theoretical maximum 100% yield. Either process mode produced a residue. When dried, the residue was a dark solid coated on the catalyst that was not soluble in water.

In semi-batch operation, the reactor was provisioned with catalyst and glycerol was fed into the reactor at a uniform rate over a period of about 1.25 hours. In batch operation, all of the glycerol and catalyst was loaded into the reactor at the start of the reaction. The following results show the semi-batch reactive-distillation has higher yields and selectivities than batch. The higher catalyst loading provided higher yields and selectivities. It was observed that the catalyst activity decreased with reaction time and the amount of residue increased with reaction time.

The copper chromium catalysts used in this Illustrative Example were used in the condition in which they arrived.

| **Table 8.** Comparison of Semi-Batch (Continuous) Reactive-distillation and Batch Reactive-distillation. Formation and isolation of acetol intermediate from glycerol using copper-chromite catalyst. Catalyst - 5% unreduced copper chromium powder |
|---|
| **Reaction conditions:** |
| Reaction Pressure-98 kPa (vac) |
| Reaction temperature-240°C |
| Reaction complete time-2 hr |
| Glycerol feed rate-33.33 g/hr for Semi-Batch Reactions |

| **The following three reactions were conducted:** |
|---|
| RXN 8.1 - Semi-Batch reaction at 5% catalyst loading |
| RXN 8.2 - Semi-Batch reaction at 2.5% catalyst loading |
| RXN 8.3 - Batch reaction 5% catalyst loading |

The following are reaction details of RXN 8.1: Initial loading of glycerol, 54.29; Glycerol in Distillate, 4.91; Residue, 3.80; and Amount of glycerol reacted, 49.38 all in grams. The glycerol reacted as described in Table 9.

| **Table 9.** Mass balance details on RXN 8.1. Catalyst loading was 5%. | | | |
|---|---|---|---|
| | Reacted Glycerol (g) | Best possible (g) | Distillate (g) |
| Glycerol | 49.38 | 0 | 3.64 |
| Acetol | 0 | 39.71 | 35.99 |
| Propylene glycol | 0 | 0 | 1.65 |
| Water | 0 | 9.66 | 5.79 |

The following are reaction details of RXN 8.2: Initial loading of glycerol, 52.8; Glycerol in Distillate, 3.85; Residue, 4.91; and Amount of glycerol reacted, 48.95 all in grams. The glycerol reacted as described in Table 10.

| **Table 10.** Mass balance details on RXN 8.2. Catalyst Ioading was 2.5%. | | | |
|---|---|---|---|
| | Reacted Glycerol (g) | Best possible (g) | Distillate (g) |
| Glycerol | 48.95 | 0 | 3.85 |
| Acetol | 0 | 39.37 | 33.51 |
| Propylene glycol | 0 | 0 | 1.63 |
| water | 0 | 9.58 | 6.24 |

The following are reaction details of RXN 8.2: Initial loading of glycerol, 42.48; Glycerol in Distillate, 3.64; Residue, 5.68; and Amount of glycerol reacted, 33.16 all in grams. The glycerol reacted as described in Table 11.

| **Table 11.** Mass balance details on RXN 8.3. Catalyst loading was 5%. | | | |
|---|---|---|---|
| | Reacted Glycerol (g) | Best possible (g) | Distillate (g) |
| Glycerol | 36.80 | 0 | 3.64 |
| Acetol | 0 | 29.60 | 23.73 |
| Propylene glycol | 0 | 0 | 1.67 |
| water | 0 | 7.2 | 6.99 |

As reported in the following examples, various studies were performed to assess the ability to control the residue problem.

### COMPARATIVE EXAMPLE 6

### Control of Residue By Water Content of Feedstock

Glycerol was reacted in presence of copper chromium catalyst to form acetol at conditions similar to Illustrative Example 4 with 2.5% catalyst loading and in a semi-batch reactor method. Water was added to the glycerol to evaluate if water would decrease the accumulation of the water-insoluble residue. Table 12 summarizes the conversion results. These data illustrate that a small amount of water reduces the tendency for residue to form. The copper chromium catalyst used in this Illustrative Example was used in the condition in which they arrived.

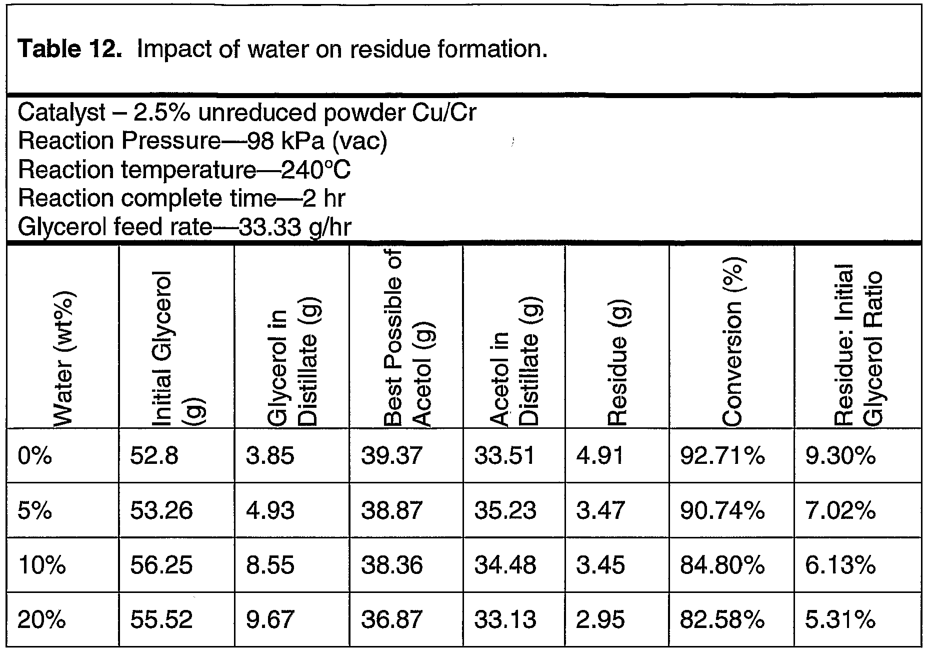

### COMPARATIVE EXAMPLE 7

### Control of Residue By Catalyst Loading

Glycerol was reacted in presence of copper chromium catalyst to form acetol in a semi-batch reactor method. The impact of lowering catalyst loadings was evaluated to determine the impact of catalyst loading on acetol yield and residue formation. Table 13 summarizes the conversion results. These data illustrate that the formation of residue may be autocatalytic-it increases more than linearly with increasing throughput of glycerol over the catalyst. Also, the selectivity decreases with increasing throughput of glycerol over a fixed catalyst loading in the reactor.

The copper chromium catalyst used in this Illustrative Example was used in the condition in which they arrived.

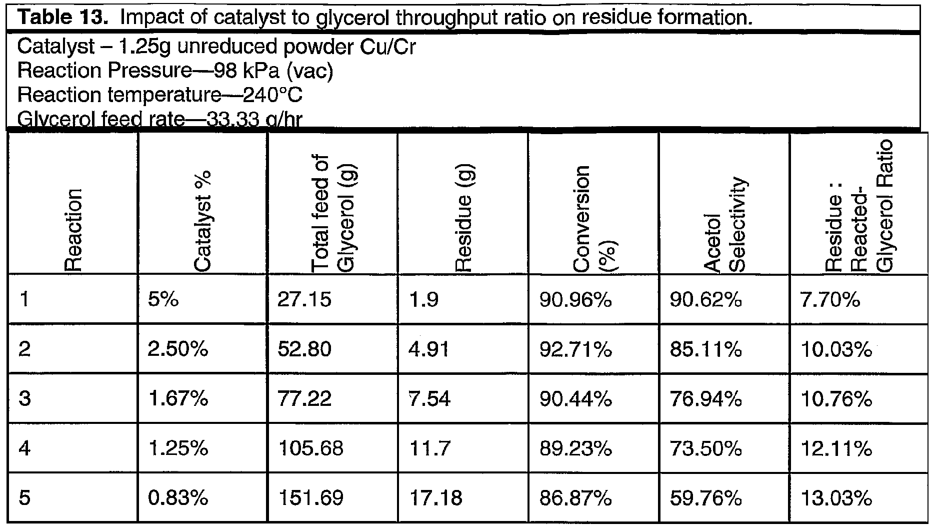

### COMPARATIVE EXAMPLE 8

### Regeneration of Catalyst

This example illustrates the stability of the copper chromium catalyst for the formation of propylene glycol from acetol. The following were the approximate initial conditions: 4.5 grams of acetol, 2 wt% of catalyst, and a hydrogen overpressure of 1400 kPa. The following table presents compositions after reacting in a closed reactor (with topping off of hydrogen) for 4 hours at a reaction temperature of 185°C. The copper chromium catalyst was reduced in presence of hydrogen at a temperature of 300°C for 4 hours prior to the reaction. The catalyst after each run was filtered from the reaction products, washed with methanol and then dried in a furnace at temperature of 80°C. This regenerated catalyst was reused in the subsequent reactions. Similar regeneration procedure is repeated 10 times and the results are summarized in Table 14. These data illustrate the ability to reuse catalyst for the hydrogenation of acetol.

The copper chromium catalyst used in this Illustrative Example was reduced in presence of hydrogen at a temperature of 300°C for 4 hours prior to the reaction.

| **Table 14:** Summary of catalyst performances based on 4.5 grams of acetol. | | | |
|---|---|---|---|
| | Acetol (g) | Propylene glycol (g) | Lactaldehyde (g) |
| initial | 4.5 | 0 | 0 |
| Run 1 | 0.5 | 3.62 | 0.51 |
| Run 2 | 0.29 | 3.85 | 0.56 |
| Run 3 | 0.19 | 4.19 | 0.53 |
| Run 4 | 0.07 | 4.41 | 0.47 |
| Run 5 | 0.05 | 4.42 | 0.49 |
| Run 6 | 0.05 | 4.39 | 0.51 |
| Run 7 | 0 | 4.41 | 0.36 |
| Run 8 | 0.24 | 4.2 | 0.42 |
| Run 9 | 0.27 | 4.2 | 0.43 |
| Run 10 | 0.21 | 4.11 | 0.4 |

### COMPARATIVE EXAMPLE 9

### Ability to Reuse Catalyst of Acetol-Forming Reaction

This example illustrates that a powder catalysts may be treated or reactivated by hydrogen treatment, but also that one powder catalyst that contains 54% CuO and 45% Cr₂O₃ has better reuse properties than does another powder catalyst at 30 m²/g surface area, 45% CuO, 47% Cr₂O₃, 3.5% MnO₂ and 2.7% BaO. For the powder catalyst at 54% CuO and 45% Cr₂O₃, the data of Table 15 demonstrate that residue formation rate is similar to that of the powder catalyst at 30 m²/g surface area, 45% CuO, 47% Cr₂O₃, 3.5% MnO₂ and 2.7% BaO (Table 14). The data of Table 16 demonstrate the 54% CuO and 45% Cr₂O₃ catalyst can be used repeatedly (at laboratory scale, 1-3% of the catalyst was not recovered from reaction to reaction). The data of Table 17 demonstrate that reuse is more difficult with the 45% CuO, 47% O₂O₃, 3.5% MnO₂ and 2.7% BaO Catalyst.

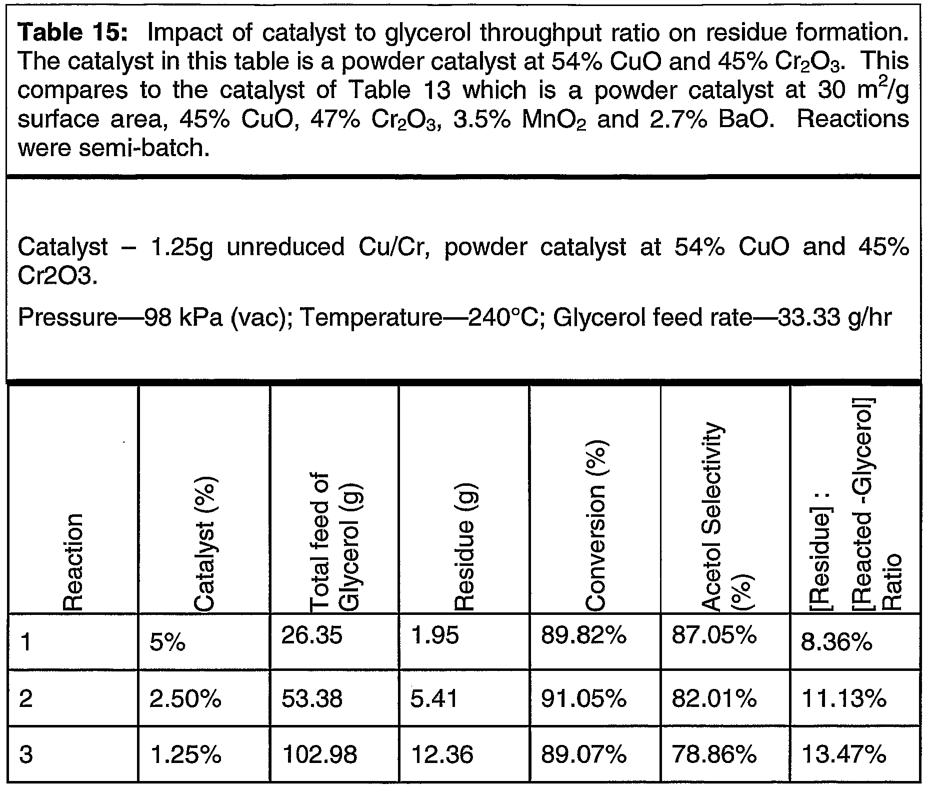

| **Table 16:** Impact of reuse on powder catalyst at 54% CuO and 45%. Catalyst is loaded at 5% and is unreduced. | | | | | |
|---|---|---|---|---|---|
| Catalyst - 2.5g unreduced Cu/Cr, powder catalyst at 54% CuO and 45% Cr₂O₃. | | | | | |
| Pressure-98 kPa (vac); Temperature-240°C; Glycerol feed rate-33.33 g/hr | | | | | |
| | Total feed of Glycerol (g) | Residue (g) | Conversio n (%) | Acetol Selectivity (%) | Residue: Initial-Glycerol Ratio |
| Fresh | 52.77 | 3.96 | 89.82% | 87.05% | 7.51% |
| Reused 1 | 52.16 | 4.11 | 91.28% | 88.52% | 7.88% |
| Reused 2 | 51.72 | 3.89 | 91.74% | 88.56% | 7.53% |
| Reused 3 | Catalysts still could be recovered | | | | |

| **Table 17:** Impact of reuse on powder catalyst at 30 m²/g surface area, 45% CuO, 47% CrgO₂, 3.5% MnO₂ and 2.7% BaO. | | | | | |
|---|---|---|---|---|---|
| Catalyst - 2.5g unreduced powder Cu/Cr | | | | | |
| Pressure-98 kPa (vac); Temperature-240°C; Glycerol feed rate-33.33 g/hr | | | | | |
| | Total feed of Glycerol (g) | Residue (g) | Conversion (%) | Acetol Selectivity (%) | Residue: Initial-Glycerol Ratio |
| Fresh | 54.29 | 3.80 | 90.95% | 90.62% | 7.01% |
| Reused 1 | 53.13 | 3.99 | 88.92% | 88.80% | 7.51% |
| Reused 2 | Catalyst could not be recovered-residue was totally solidified | | | | |

The two catalysts at initial condition performed about the same for the acetol forming reaction; however, .the 45% CuO, 47% Cr₂O₃, 3.5% MnO₂ and 2.7% BaO catalyst at a loading of lesser than 5% formed a different type of residue that was more resistant to catalyst recovery. For both catalysts, it was generally observed that as reactions proceeded, the reaction rates tended to reduce. At the end of the semi-batch reaction a digestion of the mixture was induced by stopping the feed and allowing the reaction to proceed for about 30 min to an hour-during this digestion the volume of the reaction mixture decreased and the residue became more apparent.

For the 54% CuO and 45% Cr₂O₃ catalyst, the residue tends to be stable. This residue takes a solid form in room temperature and a slurry form at the reaction temperature during the long period of reaction time. A methanol wash readily removed the residue, allowing the catalyst to be reused multiple times. The solid was soft and tacky in nature and readily dissolved in methanol to form slurry. The catalyst was washed with methanol until the wash was clear and then the catalyst was dried in a furnace at 80° C to remove the methanol. The physical appearance of this catalyst after washing was similar to that of the new catalyst.

In the case of 45% CuO, 47% Cr₂O₃, 3.5% MnO₂ and 2.7% BaO catalyst the residue was, however, different. In the case of 5% catalyst loading, residue started foaming on the catalyst at 30 min after total glycerin was fed, i.e., 30 minutes into the reaction. Once foaming started, a methanol wash was not effective for removing the residue from the catalyst. If the reaction was stopped prior to commencement of foaming, the methanol was effective in removing the residue from the catalyst. When catalyst loading less than 2.5%, the residue started foaming while the glycerin was still being fed to the reactor, and the catalyst could not be recovered at end of the reaction. The 54% CuO and 45% Cr2O3 catalyst produced a residue that is a solid at room temperature.

These trends in reuse of catalyst are applicable to conditions for conversion of glycerin to acetol as well as the "single-pot" conversion of glycerin to propylene glycol.

### COMPARATIVE EXAMPLE 10

### Lactaldehyde Mechanism

Acetol was hydrogenated in presence of copper chromium catalyst to form a mixture containing propylene glycol. The following were the approximate initial conditions: 10 grams of acetol, 2 wt% of catalyst, and a hydrogen overpressure of 1400 kPa. The following table presents compositions after reacting in a closed reactor (with topping off of hydrogen) for 4 hours at a reaction temperature of 190°C. Table 18 shows the effect of reaction temperature on the formation of propylene glycol from acetol. The data illustrate that good conversions are attainable at 190°C. The data illustrate that the co-product (likely undesirable) of lactaldehyde is produced at lower selectivities at temperatures greater than 150°C. Optimal temperatures appear to be 190°C or higher. The copper chromium catalyst used in this Illustrative Example was reduced in presence of hydrogen at a temperature of 300°C for 4 hours prior to the reaction.

| **Table 18:** Summary of catalyst performances based on 9 grams of acetol. The pressure is 1400 kPa with a 5% catalyst loading. | | | |
|---|---|---|---|
| Temperature C | Acetol (g) | Propylene glycol (g) | Lactaldehyde (g) |
| Un reacted | 10 | 0 | 0 |
| 50 | 8.25 | 1.86 | 0.13 |
| 100 | 5.74 | 3.93 | 0.47 |
| 150 | 3.10 | 4.31 | 2.82 |
| 180 | 1.64 | 7.90 | 0.89 |
| 190 | 0.56 | 9.17 | 0.58 |

Table 19 shows the effect of initial water content in the reactants on the formation of propylene glycol from acetol. The data illustrate that water can improve yields to propylene glycol. Selectivity to propylene glycol decreases as the reaction goes beyond 10-12 hrs.

| **Table 19:** Summary of catalyst performances based on different initial loadings of water. The reaction temperature is 190°C, at a pressure of 1400 kPa, a 5% catalyst loading and a reaction time of 24 hours. The total loading of water with acetol is 10 grams. | | | |
|---|---|---|---|
| Water (%wt) | Acetol (g) | Propylene glycol (g) | Lactaldehyde (g) |
| 10 | 0.47 | 7.65 | 0 |
| 20 | 0.22 | 5.98 | 0.7 |
| 50 | 0.22 | 4.35 | 0.27 |

Table 20 shows the effect of initial catalyst concentration on the formation of propylene glycol from acetol. The data illustrate that the highest yields are attained at the higher catalyst loadings.

| **Table 20:** Summary of catalyst performances based on 4.5 grams of acetol. The reaction temperature is 190°C, at a pressure of 1400 kPa, and no added water. | | | | |
|---|---|---|---|---|
| Catalyst | Reaction Time (h) | Acetol (g) | Propylene glycol (g) | Lactaldeh yde (g) |
| Concentration (wt%) | | | | |
| Initial | - | 4.5 | 0 | 0 |
| 5% | 4 | 0.29 | 4.46 | 0.22 |
| 2% | 4 | 0.14 | 4.27 | 0.2 |
| 1% | 4 | 1.32 | 3.45 | 0.29 |
| 0.5% | 4 | 1.56 | 3.14 | 0.32 |
| 1% | 6 | 0.58 | 3.78 | 0.25 |
| 0.5% | 6 | 1.27 | 3.29 | 0.33 |

### Reactive-Separation With Gas Stripping According to the Present Invention

The use of the reactor-separator 102 is very effective for converting glycerol to acetol as illustrated by the foregoing Examples. These examples illustrate, for example, the effective use of water and catalyst loading to reduce formation of residue. Two disadvantages of the reactions were the formation of residual and operation at small amounts of vacuum.

The present invention overcomes the vacuum operation by using hydrogen as a stripper gas to strip the acetol from solution. Thus, the process equipment operates at a more optimal pressure, such as slightly over atmospheric pressure, to make advantageous use of fugacity (partial pressures) for the selective removal of vapor from the reaction mixture.

Use of hydrogen at pressures slightly above atmospheric pressure strips the acetol from solution as it is formed. In addition, the hydrogen stripper gas keeps the catalyst reduced and provides reaction paths that prevent residue formation and/or react with the residue to form smaller molecules that also strip from solution. The reactions that strip the residue may include use of additional catalysts that are known to be effective for catalytic cracking, and the use of such stripper gas in combination with catalytic cracking catalysts is referred to herein as a strip-crack process. The hydrogen is preferably either recycled in the reactor or compressed with the acetol for a second reaction at higher pressure.

A reaction process that may be used to perform the method includes a first reactor. In the first reactor, the first product and alternative product are removed as vapor effluents from a liquid reaction where a sufficient hydrogen pressure is present to reduce the residue formation by at least 50% as compared to the residue formation rate without hydrogen present. The preferred hydrogen partial pressures are between 0.2 and 50 bars, more preferably between 0.5 and 30 bars, and most preferably between 0.8 and 5 bars.

To achieve higher conversions to the alternative product, the first product may be reacted in a second reactor that is operated at higher partial pressures of hydrogen. In the second reactor, the partial pressure of hydrogen is at least twice the partial pressure of hydrogen in the second reactor, more preferably the partial pressure of hydrogen is at least four times the partial pressure of hydrogen in the first reactor.

The respective temperatures of the first and second reactors are preferably above the normal boiling point of the first product.

The use of hydrogen has an additional advantage of reducing residue that tends to deactivate catalysts which are useful in the disclosed process. In this sense, the hydrogen may be used as the gas purge or stripper gas, as well as a reagent in the first reactor. For example, in the cracking of petroleum to gasoline, it is well-known that hydrogen reduces the formation of residue that tends to deactivate the catalyst; however, the use of hydrogen is more expensive than cracking of petroleum in the absence of hydrogen. In petroleum industry practice, considerable catalytic cracking is performed in the absence of hydrogen with product loss, and specialized equipment is devoted to regenerating the deactivated catalyst. Those other practices differ from the presently disclosed use of hydrogen stripper gas that is sufficient to reduce catalyst deactivation, but is sufficiently low in amount and amount/pressure to allow the non-hydrogen cat-cracking to dominate, e.g., at a pressure less that 50 bars, while the reaction is underway.

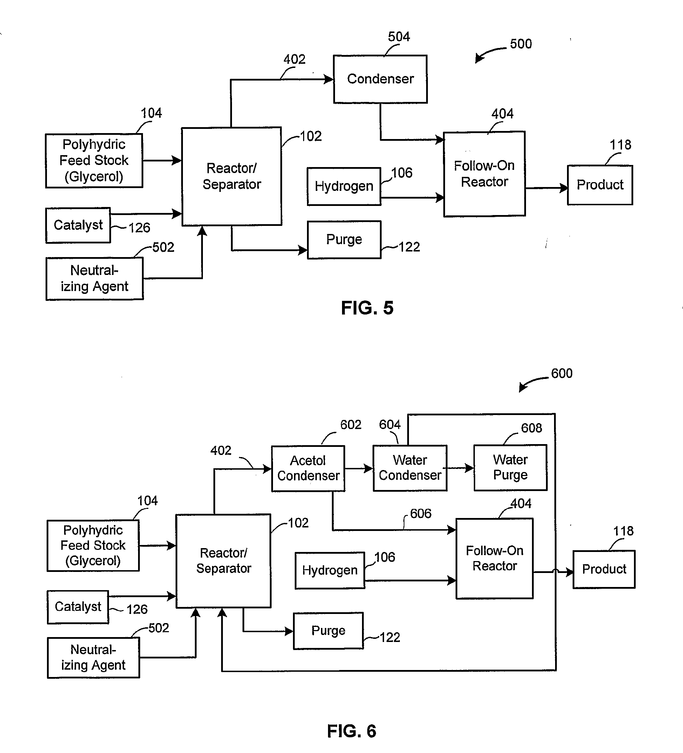

Fig. 5 shows one embodiment that implements these concepts. Process equipment 500 was used in the process where the hydrogen is compressed to proceed to the second reaction. In Fig. 5, like numbering is maintained with respect to identical elements as shown in Fig. 4. The reaction process proceeds as described with respect to Fig. 4, except low pressure hydrogen stripper gas 502 is applied to reactor separator 102, for example, at a pressure slightly above atmospheric pressure. Although some of this gas does result in the production of propylene glycol, the stripping of acetol is predominant. A mixture of acetol, propylene glycol and water vapor flows through overhead line 402 to compressor 504, which pressurizes the vapors to a suitably higher pressure for use in the follow-on reactor 404. The extant hydrogen is optionally supplemented by additional hydrogen 106 to establish the preferred reaction conditions discussed above.

Fig. 6 shows another embodiment, that of process equipment 600. In Fig. 6, like numbering is maintained with respect to identical elements as shown in Fig. 5. In process equipment 600, the effluent through intermediate overhead line 402 is applied to a series of condensers 602,604 that decrease in their relative temperatures to condense first the acetol in acetol condenser 604 and then water in water condenser 604. The condensed acetol is applied to line 606, e.g., by pumping at the requisite pressure, for delivery to the follow-on reactor 404. Water effluent from water condenser 604 is discharged as water purge 608.

The hydrogen pressures (or partial pressures) for this process in the respective reactor vessels may be lower than is required for "good" hydrocracking and/or hydrogenolysis but sufficient to stop catalyst deactivation.

In addition to reactor configurations, other methods known in the science for reducing residue (often an oligomer) formation is the use of a solvent. The solvent can reduce residue formation or dissolve the residue therein extending catalyst life. Solvents are preferably not reactive liquids. Supercritical solvents, such as carbon dioxide, have also been demonstrated as effective for extending catalyst life when residue formation otherwise coats the catalyst.

### Applicability to Broader Reaction Mechanisms

The process that has been shown and described has been proven effective for production of acetol and propylene glycol, but is not limited to the reaction mechanisms of Figs. 2 and 3. The process and process equipment is generally applicable to a range of reactions having similar overall mechanisms including at least four classes of such reactions in context of the discussion below.

A first class of liquid phase catalytic reaction occurs where a reactant (e.g. glycerol) distributes predominantly in a liquid phase and the reactant is converted to at least a first product (e.g. acetol) that has a boiling point at least 20° C lower in temperature than the reactant.

A second class of liquid phase catalytic reaction occurs where the reactant reacts in a parallel mechanism with hydrogen to form at least one alternative product (e.g. propylene glycol) where the alternative product has a boiling point that is at least 20° C lower in temperature than the reactant. The selectivity to formation of the alternative produce(s) from this second reaction is greater than 0.5 when in the presence of hydrogen and hydrogen partial pressures in excess of 100 bars.

A third class of reaction proceeds substantially in parallel to the first reaction including the reactant forming a higher molecular weight residue species that directly or indirectly reduces the effectiveness of the catalyst promoting the first reaction.

A fourth class of reaction that occurs when hydrogen is present that substantially inhibits the formation of the residue of the third reaction where the rate of formation of residue is reduced by at least 50% with the hydrogen partial pressure in 50 bars.

The process includes use in appropriate reactor configurations, such as the process equipment 100, 400, 500 and/or 600 discussed above.

## Claims

1. A process for converting glycerol to acetol with high selectivity, comprising the steps of:
combining a glycerol-containing feedstock with less than 50% by weight water with a catalyst that is capable of dehydrating glycerol to form a reaction mixture;
heating the reaction mixture to a temperature ranging from 170°C to 270°C over a reaction time of up to 24 hours at a pressure ranging from 0.2 to 25 bar; and
using hydrogen as a stripping gas to strip the acetol from solution.

2. The process of claim 1, wherein the glycerol-containing feedstock used in the step of combining contains from 0% to 15% water in glycerol by weight.

3. The process of claim 1, wherein the catalyst used in the step of combining is a heterogeneous catalyst selected from the group consisting of palladium, nickel, rhodium, copper, zinc, chromium and combinations thereof.

4. The process of any one of the preceding claims, wherein the temperature used in the heating step ranges from 180°C to 240°C.

5. The process of any one of the preceding claims, wherein the pressure used in the heating step ranges from 0.506625 to 3.03975 bar (0.5 to 3 atmospheres).

6. The process of claim 1, comprising a step of condensing the vapors to yield liquid reaction product.

7. The process of claim 6, wherein the step of condensing occurs using a condenser operating at a temperature ranging from 0°C to 140°C

8. The process of claim 7, wherein the condenser operates at a temperature ranging from 25°C to 60°C.

9. The process of claim 1, comprising a subsequent step of contacting the acetol with a hydrogen co-reagent to promote conversion of glycerol to propylene glycol.

## Patentansprüche

1. Verfahren zum Umwandeln von Glyzerin in Hydroxyaceton mit hoher Selektivität, das folgende Schritte umfasst:
Kombinieren eines Glyzerin enthaltenden Ausgangsstoffs mit weniger als 50 Gew.-% Wasser mit einem Katalysator, der zum Dehydratisieren von Glyzerin fähig ist, um ein Reaktionsgemisch zu bilden;
Erwärmen des Reaktionsgemisches auf eine Temperatur zwischen 170 °C und 270 °C während einer Reaktionszeit von bis zu 24 Stunden bei einem Druck zwischen 0,2 bar und 25 bar; und
Verwenden von Wasserstoff als Ablösegas, um das Hydroxyaceton von einer Lösung abzulösen.

2. Verfahren nach Anspruch 1, wobei der Glyzerin enthaltende Ausgangsstoff, der in dem Schritt des Kombinierens verwendet wird, 0 Gew.-% bis 15 Gew.-% Wasser in Glyzerin enthält.

3. Verfahren nach Anspruch 1, wobei der Katalysator, der in dem Schritt des Kombinierens verwendet wird, ein heterogener Katalysator ist, der aus der Gruppe ausgewählt wird, die Palladium, Nickel, Rhodium, Kupfer, Zink, Chrom und Kombinationen daraus umfasst.

4. Verfahren nach einem der vorhergehenden Ansprüche, wobei die Temperatur, die in dem Erwärmungsschritt verwendet wird, zwischen 180 °C und 240 °C liegt.

5. Verfahren nach einem der vorhergehenden Ansprüche, wobei der Druck, der in dem Erwärmungsschritt verwendet wird, zwischen 0,506625 bar und 3,03975 bar (0,5 Atmosphären bis 3 Atmosphären) liegt.

6. Verfahren nach Anspruch 1, das einen Schritt des Kondensierens der Dämpfe umfasst, um ein flüssiges Reaktionsprodukt zu ergeben.

7. Verfahren nach Anspruch 6, wobei der Schritt des Kondensierens unter Verwendung eines Kondensators geschieht, der bei einer Temperatur zwischen 0 °C und 140 °C arbeitet.

8. Verfahren nach Anspruch 7, wobei der Kondensator bei einer Temperatur zwischen 25 °C und 60 °C arbeitet.

9. Verfahren nach Anspruch 1, das einen nachfolgenden Schritt des Herstellens eines Kontakts zwischen Hydroxyacetons und einem Ko-Reagenz umfasst, um die Umwandlung von Glyzerin in Propylenglykol zu fördern.

## Revendications

1. Procédé de conversion de glycérol en acétol à haute sélectivité, comportant les étapes consistant à :
combiner une charge contenant du glycérol ayant moins de 50 % en poids d'eau, avec un catalyseur qui est capable de déshydrater le glycérol pour former un mélange réactionnel ;
chauffer le mélange réactionnel jusqu'à une température variant de 170 °C à 270 °C sur un temps de réaction allant jusqu'à 24 heures à une pression variant de 0,2 à 25 bar ; et
utiliser de l'hydrogène comme gaz de revaporisation pour revaporiser l'acétol à partir de la solution.

2. Procédé selon la revendication 1, dans lequel la charge contenant du glycérol utilisée à l'étape de combinaison contient de 0 % à 15 % en poids d'eau dans le glycérol.

3. Procédé selon la revendication 1, dans lequel le catalyseur utilisé à l'étape de combinaison est un catalyseur hétérogène choisi parmi le groupe constitué de palladium, de nickel, de rhodium, du cuivre, de zinc, de chrome et de combinaisons de ceux-ci.

4. Procédé selon l'une quelconque des revendications précédentes, dans lequel la température utilisée à l'étape de chauffage varie de 180 °C à 240 °C.

5. Procédé selon l'une quelconque des revendications précédentes, dans lequel la pression utilisée à l'étape de chauffage varie de 0,506625 à 3,03975 bar (0,5 à 3 atmosphères).

6. Procédé selon la revendication 1, comportant une étape de condensation des vapeurs pour produire un produit de réaction liquide.

7. Procédé selon la revendication 6, dans lequel l'étape de condensation a lieu en utilisant un condenseur fonctionnant à une température variant de 0 °C à 140 °C.

8. Procédé selon la revendication 7, dans lequel le condenseur fonctionne à une température variant de 25 °C à 60 °C.

9. Procédé selon la revendication 1, comportant une étape ultérieure consistant à mettre en contact l'acétol avec un co-réactif d'hydrogène pour favoriser la conversion du glycérol en propylène glycol.
